# FASCICULE DE BREVET EUROPEEN

(11) **EP 3 448 226 B1**
(45) Date de publication et mention de la délivrance du brevet: **25.03.2020**
(21) Numéro de dépôt: 17742465.2
(22) Date de dépôt: 06.06.2017
(51) Int. Cl.: A61B 1/00

(54) **CAPUCHON ENDOSCOPIQUE À PAROI RENFORCÉE**
ENDOSKOPISCHE KAPPE MIT VERSTÄRKTER WAND
ENDOSCOPIC CAP WITH REINFORCED WALL

(30) Priorité: 06.06.2016 FR 1655163
(43) Date de publication de la demande: 06.03.2019
(73) Titulaire: Assistance Publique-Hôpitaux de Paris (AP-HP), 75004 Paris (FR)
(72) Inventeur: VIALA, Jêrome, 95440 ECOUEN (FR)
(74) Mandataire: Novagraaf Technologies
(86) Numéro de dépôt international: PCT/FR2017/051421
(87) Numéro de publication internationale: WO 2017/212163

(56) Documents cités:
- WO-A1-2016/064385
- FR-A1- 2 977 135
- US-A1- 2014 296 768
- LE GROUPE FRANCOPHONE D'HEPATOLOGIE GASTROENTEROLOGIE ET NUTRITION ET AL: "Ingestion de corps etrangers chez l'enfant. Recommandations du Groupe francophone d'hepatologie, gastroenterologie et nutrition pediatriques", ARCHIVES DE PEDIATRIE, ELSEVIER, PARIS, FR, vol. 16, no. 1, 6 décembre 2008 (2008-12-06), pages 54-61, XP025870108, ISSN: 0929-693X, DOI: 10.1016/J.ARCPED.2008.10.018 [extrait le 2008-12-06]

## Description

### Domaine technique

La présente invention relève du domaine des accessoires d'endoscope pour l'extraction de corps étrangers d'un organisme humain ou animal.

La présente invention se rapporte notamment à un capuchon d'endoscope destiné à se fixer sur l'extrémité distale d'un endoscope en vu de couvrir l'objet destiné à être extrait.

Les références entre crochet ([..]) renvoient à la liste des références à la fin de la description.

### Art antérieur

L'ingestion de corps étrangers relève de situations très différentes chez l'enfant et chez l'adulte. Entre 1 et 3 ans, l'enfant explore son environnement extérieur par les sites les mieux innervés de son corps, ses doigts et sa bouche. Il lui est naturel d'ingérer une grande diversité d'objets. En 2007, les centres antipoison américains (« *American Poison Control Centers* ») avaient répertorié 125.000 cas d'ingestion de corps étrangers **[1].** Certains de ces objets ont un caractère dangereux par leur caractère tranchant ou coupant **[2].** Chez l'adolescent et l'adulte, es ingestions accidentelles se font plus rares et les ingestions volontaires à but d'autolyse deviennent plus fréquentes. Les objets ingérés sont alors fréquemment et volontairement traumatisants **[3].** Egalement, les patients souffrant de troubles du comportement, dus à une encéphalopathie ou un syndrome autistique, peuvent également ingérer des objets traumatisants.

Ces objets traumatisants, perforants ou tranchants peuvent causer de graves lésions du tube digestif, jusqu'à la perforation **[4].** Ces perforations se compliquent fréquemment d'infections de voisinage, médiastinite ou péritonite. S'ils traversent la paroi digestive, ces corps étrangers traumatisants peuvent léser d'autres organes, tels que les poumons, le foie, la rate ou déclencher d'importantes hémorragies par lésion des gros vaisseaux. L'œsophage est l'organe le plus fréquemment lésé du fait de son diamètre réduit, de sa contractilité et de la faible épaisseur de sa paroi.

Plusieurs recommandations internationales exigent une extraction endoscopique de ces corps étrangers traumatisants le plus rapidement possible **[2], [5].** En effet, s'ils devaient franchir l'angle de Treitz, ils deviendraient inaccessibles à l'endoscopie digestive et risqueraient de traumatiser l'intestin grêle. Le risque de perforation a été estimé à 4,8% dans une étude adulte rétrospective **[6].** Cependant, l'extraction endoscopique n'est pas sans risque puisque de passage retour du corps étranger par l'œsophage peut être responsable de nouvelles lésions œsophagiennes du fait de la traction endoscopique.

Une solution connue pour limiter le risque de lésion œsophagienne lors du retrait, est d'employer un surtube endoscopique. Le surtube consiste à tunneliser l'œsophage avec un tube de plastique dont le diamètre interne accepte le passage de l'endoscope **[3].** Cependant, son diamètre le rend inutilisable chez le jeune enfant.

Une autre solution connue, décrit dans WO 2016/064385, est d'employer un capuchon endoscopique (encore appelé « protection hood » en langue anglaise). Ce type de capuchon est en plastique souple et est fixé à l'extrémité de l'endoscope à l'aide d'un élastique **[7].** Cependant, ce matériel est peu résistant à la perforation ou à la lacération. De plus, son repliement en avant de l'extrémité de l'endoscope réduit grandement le champ de vision, rendant difficile la préhension du corps étranger. Il peut même camoufler le fait que ce dernier a transpercé le capuchon. L'extraction risque alors de léser l'œsophage sans que l'endoscopiste ne puisse s'en rendre compte, favorisant aussi une lésion plus étendue.

A noter que ce type de difficultés n'est pas propre aux humains, mais peux également se rencontrer chez les animaux ayant avalé un corps étranger perforant ou sectionnant, que ce soit accidentellement ou suite à une action d'un individu mal intentionné.

Par ailleurs, de telles difficultés peuvent se rencontrer dès lors qu'il s'agit de retirer un corps étranger d'une cavité d'un organisme autre que l'estomac sans endommager les tissus lors du retrait de l'endoscope.

Le problème que vise à résoudre la présente invention est donc, dans le cadre du retrait d'un corps étranger coupant ou perforant situé à l'intérieur de la cavité d'un organisme humain ou animal, de diminuer le risque de lésion des tissus lors du retrait d'un endoscope après que ce dernier ait saisi le corps étranger.

### Exposé de l'invention

Ce problème est résolu par l'objet de la présente invention.

L'objet de la présente invention est donc un capuchon d'endoscope comprenant :
- une enveloppe de protection formée par une paroi repliée sur elle-même de manière à ce que ladite enveloppe comprenne une ouverture proximale et une ouverture distale en vis-à-vis de l'ouverture proximale et présente une forme s'évasant depuis son ouverture proximale vers son ouverture distale, selon une configuration de base,
- un moyen de fixation agencé de manière à permettre la fixation du capuchon sur l'extrémité distale d'un endoscope avec ladite ouverture proximale en vis-à-vis ou autour de l'extrémité distale de l'endoscope,
la paroi de ladite enveloppe étant réalisée en une structure présentant une résistance suffisante pour résister à une perforation et/ou à une incision par un objet pointu et/ou tranchant.

Ainsi les inventeurs ont choisi de conserver l'utilisation d'un capuchon mais en travaillant sur la structure de ses parois. Ils se sont aperçu qu'ils pouvaient ainsi obtenir de bons résultats en protégeant les tissus lors du retrait de l'endoscope.

Ce capuchon peut ainsi se fixer à l'extrémité de l'endoscope qui est introduite dans l'organisme, extrémité appelée extrémité distale de l'endoscope.

Le moyen de fixation permet soit le positionnement de l'ouverture proximale en face de l'extrémité distale de l'endoscope, soit à l'endoscope de passer au travers de cette ouverture proximale. La vision au moyen de l'endoscope au-delà de cette ouverture proximale est donc possible.

L'ouverture distale du capuchon, qui est donc à l'opposé de l'ouverture proximale dans le capuchon, est la partie la plus éloignée de l'endoscope. En étant en vis-à-vis de l'ouverture proximale, cela permet donc également la vision au moyen de l'endoscope au-delà de cette ouverture distale, même en configuration de base, facilitant ainsi la recherche du corps étranger.

La configuration de base est la configuration qui sera utilisée lors du retour de l'endoscope hors de l'organisme. La forme évasée du capuchon dans cette configuration de base permet d'envelopper le corps étranger et ainsi de protéger les tissus lors de ce passage retour.

Le capuchon selon l'invention peut optionnellement comprendre une ou plusieurs des caractéristiques suivantes :
- l'enveloppe peut être sous forme d'une cloche, l'ouverture proximale étant agencée au sommet de cette cloche, et l'ouverture distale étant agencée à la base de cette cloche ; par le terme « cloche », on entend dans la présente demande toute forme s'évasant de l'ouverture proximale à l'ouverture distale, ce qui inclut notamment des formes symétriques de révolution avec une génératrice arquée ou des formes coniques ; cette forme de cloche facilite la visualisation, l'approche, la préhension et l'enveloppement du corps étranger ;
- dans sa configuration de base, l'enveloppe définit un logement et la paroi de ladite enveloppe peut comprendre un renfort agencé de manière à entourer ce logement ; ce renfort permet une réalisation simple permettant de prévenir tout risque de perforation ou incision du capuchon par le corps étranger ; par ailleurs, lors de la recherche du corps étranger, ce renfort permet de maintenir plus fermement l'ouverture distale ouverte au maximum de son diamètre, sans repliement, et en vis-à-vis de l'ouverture proximale, facilitant ainsi la vision ;
- le renfort peut être formé d'un ou plusieurs fils ; c'est une forme simple de réalisation d'un renfort ;
- le renfort, notamment le ou les fils, peuvent être formés d'un matériau métallique ou carboné ; ces matériaux présentent une bonne résistance à l'incision, tout en présentant une certaine souplesse, améliorant donc ainsi également le passage du capuchon dans l'organisme et l'enveloppement du corps étranger ;
- le renfort peut être formé par au moins un élément choisi parmi :
   o un ou des fils métalliques,
   o un ou des fils utilisés pour réaliser des endoprothèses tubulaires,
   o un ensemble de fibres, notamment de fibres de carbone ;
   ces matériaux permettent de réaliser un renfort présentant une très bonne résistance à l'incision ;
- le renfort peut être formé par un ou des fils constitués d'un matériau à mémoire de forme, notamment un alliage de nickel et de titane, notamment du nitinol ; cela permet de conférer une forme au capuchon par déformation de sa configuration de base pour faciliter son introduction dans l'organisme ; une fois dans la cavité, le capuchon est exposé à la chaleur de l'organisme et reprend sa configuration de base ;
- le ou les fils peuvent former un maillage ; cela permet d'améliorer la résistance du capuchon à l'incision ; par exemple, ce maillage peut comprendre des mailles d'environ 1 mm² de côté ;
- l'enveloppe peut comprendre au moins une couche composée d'au moins un polymère de recouvrement externe recouvrant le renfort à l'extérieur dudit logement ; cela permet de protéger les tissus lors de l'introduction et/ou le retrait de l'endoscope ;
- l'enveloppe peut comprendre une autre couche composée d'au moins un polymère de recouvrement interne recouvrant le renfort à l'intérieur dudit logement ; cela permet de renforcer la résistance à la perforation du capuchon ; par ailleurs, lorsque le capuchon est conçu pour se retourner sur lui-même pour être introduit dans l'organisme, cela permet également de protéger les tissus ;
- le polymère de recouvrement interne et le polymère de recouvrement externe peuvent être identiques ;
- la paroi de l'enveloppe peut être formée uniquement d'un ou plusieurs polymères ; dans ce cas elle peut être dépourvue de renfort ; il s'agit de polymères présentant une résistance suffisante pour résister à une perforation et/ou à une incision par un objet pointu et/ou tranchant ; ces polymères peuvent être notamment renforcés par des charges ;
- le moyen de fixation peut comprendre un manchon de rétention présentant une première extrémité destinée à se fixer sur l'extrémité distale de l'endoscope, le manchon de rétention présentant une deuxième extrémité fixée sur l'enveloppe de manière à ce que le manchon débouche dans l'ouverture proximale ; ce manchon forme un moyen simple de fixation du capuchon, tout en permettant de positionner l'ouverture proximale pour permettre la vision au-delà de celle-ci ;
- le manchon et la paroi de ladite enveloppe peuvent être venus de matière en une seule pièce ; notamment ils peuvent être obtenus en étant moulés ensemble ou en moulant ensemble le manchon et une couche de recouvrement du renfort ;
- le manchon peut être constitué d'un matériau composé d'un ou plusieurs polymères, dits polymères du manchon ;
- le matériau constituant le manchon peut être élastique et/ou poreux ; cela permet de l'enfiler plus facilement sur l'extrémité distale de l'endoscope et augmente son adhésion sur cette dernière ;
- le manchon peut présenter une surface interne comprenant au moins un creux et/ou au moins un relief ; cela augmente l'adhésion du manchon sur l'extrémité distale de l'endoscope, notamment lorsque l'endoscope comprend une forme complémentaire de ce creux ou de ce relief ;
- la paroi peut présenter une souplesse permettant à l'enveloppe d'être mise dans une configuration repliée, dans laquelle l'enveloppe est retournée sur elle-même autour du moyen de fixation, et de passer par déroulement de cette configuration à une configuration dépliée, correspondant à la configuration de base et dans laquelle ladite enveloppe est retournée dans l'autre sens de sorte qu'elle est agencée à côté du moyen de fixation ; une fois le capuchon sur l'endoscope, on peut retourner celui-ci pour dégager davantage le champs de vision en avant de l'endoscope ;
- selon l'alinéa précédent et lorsque l'enveloppe comprend un renfort, le renfort peut ne débuter qu'au niveau du sommet de l'enveloppe en étant exclusivement dans l'enveloppe, le moyen de fixation en étant dépourvu, la zone adjacente au renfort étant ainsi plus souple que la paroi de l'enveloppe et formant de ce fait une articulation autour de laquelle l'enveloppe est apte à être retournée sur elle-même dans sa configuration repliée ;
- l'enveloppe peut être agencée de manière à pouvoir passer, par déformation, d'une configuration compactée à une configuration déployée, correspondant à la configuration de base, l'enveloppe présentant dans sa configuration compactée un évasement réduit de l'enveloppe par rapport à la configuration de base ou étant dépourvue d'évasement, tout en conservant l'ouverture distale en vis à vis de l'ouverture proximale ; cela permet d'introduire plus facilement le capuchon sans le retourner, tout en conservant une bonne visibilité devant l'endoscope,
- selon l'invention, dans la configuration compactée, l'enveloppe peut présenter des portions de repli repliées sur elles-mêmes, selon des plis s'étendant longitudinalement de l'ouverture distale vers l'ouverture proximale, et dans lequel les portions de repli sont dépliées en configuration déployée ;
- le capuchon peut comprendre un renfort en matériau à mémoire de forme dont la température de fin de passage en phase austénitique est comprise entre 34 et 42 degrés, l'enveloppe étant agencée de manière à présenter la configuration déployée en phase austénitique de ce matériau ; cela permet de réaliser simplement le capuchon, d'en faciliter la déformation pour son introduction dans l'organisme, ainsi que le déploiement du capuchon, qui se fait donc sans action de l'endoscopiste ;
- la paroi de l'enveloppe peut présenter une élasticité telle, qu'en configuration compactée, cette paroi subit une contrainte générant une force de rappel tendant à la faire évoluer de sa configuration compactée à sa configuration déployée, le capuchon comprenant un ou des éléments de maintien de l'enveloppe en configuration compactée ; le déploiement de l'enveloppe peut ainsi être réalisé par l'actionnement de ce ou ces éléments de maintien par un actionneur de l'endoscope ; ce ou ces éléments de maintien peuvent être un ou des fils cerclant l'enveloppe en configuration compactée ;
- la paroi formant l'enveloppe peut être discontinue et fendue sur toute sa longueur, d'une extrémité proximale à une extrémité distale, de manière à ce que l'enveloppe puisse être obtenue par enroulement de la paroi sur elle-même dans le sens de la longueur, de manière à fermer l'enveloppe entre l'extrémité proximale, destinée à être autour de l'endoscope, et l'extrémité distale, qui forme ainsi les bords de l'ouverture distale ; on a ainsi un dispositif facilement adaptable à divers endoscopes ;
- l'enveloppe peut présenter des dimensions de manière à ce que son logement comprenne un volume d'environ 4 centimètres (cm) de diamètre et de 5 à 6 cm de long ; l'ouverture distale peut présenter un diamètre d'au moins 4 cm.

L'invention a également pour objet un endoscope équipé d'un capuchon selon l'invention, ce capuchon étant fixé sur l'extrémité distale de l'endoscope par le moyen de fixation du capuchon avec l'ouverture proximale du capuchon en vis-à-vis ou autour de l'extrémité distale de l'endoscope. L'endoscope ainsi équipé peut comprendre un élastique entourant le capuchon et l'extrémité distale de l'endoscope en étant serré de manière à maintenir ou à compléter le maintien du capuchon sur l'extrémité distale.

L'invention a également pour objet un procédé de fabrication d'un procédé de fabrication d'un capuchon d'endoscope selon l'invention, le capuchon comprenant une enveloppe comprenant une ouverture proximale et une ouverture distale en vis-à-vis de l'ouverture proximale et présentant une forme d'une configuration de base, cette forme s'évasant depuis son ouverture proximale vers son ouverture distale, ledit procédé de fabrication, comprenant:
- une étape de réalisation et/ou de conformation d'un renfort soit en forme de manche dont la forme correspond à au moins une partie de la forme de configuration de base, soit en forme de cape,
- une étape de recouvrement du renfort par une couche composée d'au moins un polymère de recouvrement sur au moins l'une des faces du renfort,
le matériau du renfort et/ou le ou les polymères de recouvrement étant résistant(s) à une perforation et/ou à une incision par un objet pointu et/ou tranchant, ledit procédé de fabrication.

Cela permet au renfort de mémoriser la forme adaptée à la configuration de base. Ainsi, le capuchon, qui comprendra ce renfort, pourra être déformé aisément avant introduction dans l'organisme et se déploiera une fois dans la cavité.

Selon le procédé de fabrication selon l'invention, le matériau du renfort peut être choisi parmi des matériaux à mémoire de forme dont la température de fin de passage en phase austénitique est comprise entre 34 et 42 degrés. L'étape de conformation du renfort est réalisée avant la mise en œuvre de l'étape de recouvrement à une température suffisante pour que cette forme devienne la forme mémorisée par le renfort, cette forme étant celle correspondant à la forme que le renfort est destiné à avoir dans la configuration de base du capuchon, le procédé de fabrication comprenant de plus :
- une étape de refroidissement du renfort en dessous de la température de fin de passage en phase martensitique du matériau du renfort,
- une étape de compactage du renfort par réduction de l'évasement de l'enveloppe par déformation du renfort, tout en conservant l'ouverture distale en vis à vis de l'ouverture proximale, cette étape de compactage étant réalisée en dessous de la température de fin de passage en phase martensitique du matériau du renfort et avant ou après l'étape de recouvrement.

Le capuchon est ainsi prêt à l'introduction avant d'être utilisé pour l'extraction.

L'étape de recouvrement peut être réalisée :
- par collage et/ou par soudage d'une paroi en matériau(x) polymère(s) sur le renfort,
- par surmoulage d'un ou plusieurs matériaux polymères sur ou autour du renfort,
- par trempage dans un matériau polymérisable, de manière à ce que le matériau polymérise autour du renfort.

L'invention a également pour objet une utilisation du capuchon selon l'invention dans un procédé d'extraction d'un corps étranger d'une cavité interne d'un organisme humain ou animal, comprenant :
- une étape de prise en main d'un endoscope équipé du capuchon, le capuchon étant fixé sur l'extrémité distale de l'endoscope avec l'ouverture proximale de ladite enveloppe en vis-à-vis de l'extrémité distale de l'endoscope,
- une étape de pénétration dans ledit organisme jusqu'à l'intérieur de ladite cavité via un orifice de cette cavité,
- une étape de saisie du corps étranger par un moyen de saisie de l'endoscope,
- une étape d'enveloppement du corps étranger par l'enveloppe du capuchon,
- une étape de retrait de l'endoscope de l'organisme.

Cette utilisation permet une extraction d'un corps étranger perforant et/ou tranchant d'un organisme sans léser les tissus, notamment les bords de l'orifice de la cavité et ceux d'un passage menant à cette cavité.

Le moyen de saisie peut notamment être une pince, un crochet, un bras de préhension.

L'utilisation selon l'invention peut optionnellement comprendre une ou plusieurs des caractéristiques suivantes :
- le capuchon peut être apte à présenter une configuration repliée et une configuration dépliée, ladite étape de pénétration étant réalisée avec le capuchon dans sa position repliée, ladite utilisation comprenant une étape de dépliement de l'enveloppe de sa configuration repliée à sa configuration dépliée, ladite étape de dépliement étant exécutée avant ou pendant ladite étape de retrait, avant que le corps étranger ne commence à sortir de ladite cavité ;
- selon l'invention, l'utilisation peut présenter les caractéristiques suivantes :
   o dans sa configuration repliée, l'enveloppe du capuchon est retournée autour du moyen de fixation et remonte le long de l'endoscope depuis l'extrémité distale de celui-ci,
   o dans sa configuration dépliée, l'enveloppe est déroulée en avant de l'extrémité de l'endoscope,
   o l'enveloppe passe de sa configuration repliée à sa configuration dépliée par déroulement de l'enveloppe,
- l'étape de dépliement peut être réalisée après l'étape de saisie du corps étranger ;
- l'étape d'enveloppement du corps étranger peut être réalisée par l'étape de dépliement, ladite enveloppe passant de sa configuration repliée à sa configuration dépliée en se déroulant autour du corps étranger ;
- l'étape de dépliement de l'enveloppe du capuchon peut être réalisée lors de l'étape de retrait, l'ouverture distale de l'enveloppe appuyant sur les bords de l'orifice de la cavité, de sorte que la poursuite du retrait entraine le déroulement de l'enveloppe ;
- selon l'invention, l'utilisation peut présenter les caractéristiques suivantes :
   o dans sa configuration repliée, l'enveloppe du capuchon est en avant de l'extrémité distale de l'endoscope et est sous une forme compactée, l'ouverture distale et l'ouverture proximale étant en vis-à-vis l'une de l'autre et ouvertes,
   o dans sa configuration dépliée l'enveloppe est plus large et s'évase de l'ouverture proximale vers l'ouverture distale,
   o l'enveloppe passe de sa configuration repliée à sa configuration dépliée par dépliement de l'enveloppe ;
- l'étape de dépliement peut être réalisée avant l'étape de saisie du corps étranger ;
- la paroi peut présenter une élasticité de manière à ce qu'en configuration repliée, la paroi subisse une contrainte générant une force de rappel tendant à la faire évoluer de sa configuration repliée à sa configuration dépliée, l'enveloppe étant maintenue sous cette contrainte par un ou des éléments de maintien, l'étape de dépliement comprenant une sous-étape d'actionnement de ce ou ces éléments de maintien par un actionneur de l'endoscope de manière à supprimer le maintien en contrainte de l'enveloppe, de sorte que l'enveloppe passe en position dépliée ;
- l'actionneur peut comprendre un fil ou un câble passant par un canal opérateur de l'endoscope et relié à une extrémité à ou aux éléments de maintien et à l'autre extrémité à une interface utilisateur permettant l'actionnement du fil ou du câble, l'actionnement du ou des éléments de maintien se faisant par actionnement du fil ou du câble ;
- l'utilisation peut comprendre une étape de fixation du capuchon sur l'extrémité distale de l'endoscope de manière à ce que cette extrémité distale, l'ouverture proximale et l'ouverture distale soient alignées ;
- l'étape de fixation peut comprendre l'emmanchement d'un manchon de rétention du capuchon sur l'extrémité distale de l'endoscope ;
- l'étape de fixation peut comprendre le placement de l'extrémité distale de l'endoscope dans un manchon de rétention du capuchon et la mise en place d'un élastique autour du manchon de rétention de manière à le maintenir sur l'endoscope ;
- l'étape de fixation peut comprendre :
   o le placement de la paroi du capuchon autour de l'extrémité distale de l'endoscope,
   o l'ajustement du capuchon le long de l'endoscope,
   o la mise en place d'un élastique sur la partie du capuchon entourant cette extrémité distale, de manière à maintenir le capuchon sur l'endoscope, l'élastique définissant un rétrécissement séparant d'un côté le manchon de rétention et de l'autre un logement en avant de l'extrémité distale.

D'autres avantages pourront encore apparaître à l'homme du métier à la lecture des exemples, illustrés par les figures annexées, donnés à titre illustratif.

### Brève description des figures

- La figure 1 représente un capuchon selon l'invention dans sa configuration de base.
- La figure 2 représente un capuchon selon l'invention, notamment selon la figure 1, dans une configuration repliée par retournement.
- La figure 3 représente un capuchon selon l'invention dans une configuration repliée par compactage.
- La figure 4 représente un capuchon selon l'invention avec un renfort interne.
- La figure 5 représente un endoscope.
- La figure 6 représente une vue de face de l'extrémité distale de l'endoscope de la figure 5.
- La figure 7 représente une utilisation de l'endoscope avec un capuchon selon l'invention.
- La figure 8 représente une autre utilisation de l'endoscope avec un capuchon selon l'invention.
- Les figures 9a et 9b représentent un capuchon selon l'invention, pourvu d'une fente et monté sur des endoscopes d'extrémités distales de diamètres différents.
- La figure 10 représente un capuchon selon l'invention formé par enroulement longitudinal.

### Description d'exemples de capuchon et de mise en œuvre

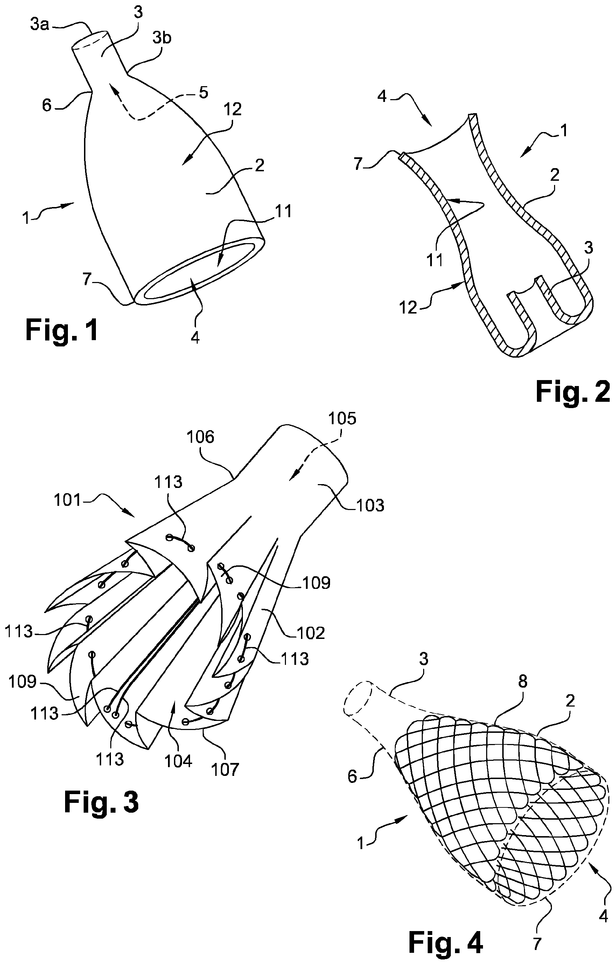

La figure 1 illustre un schéma d'un exemple de capuchon 1 selon l'invention, selon une configuration dite configuration de base.

Celui-ci comprend une enveloppe 2 de protection formée par une paroi repliée sur elle-même de manière à ce que cette enveloppe comprenne une ouverture proximale 5 et une ouverture distale 4. En d'autre terme, l'enveloppe forme une nasse avec un sommet 6 comprenant l'ouverture proximale 5 et une base 7 comprenant l'ouverture distale 4.

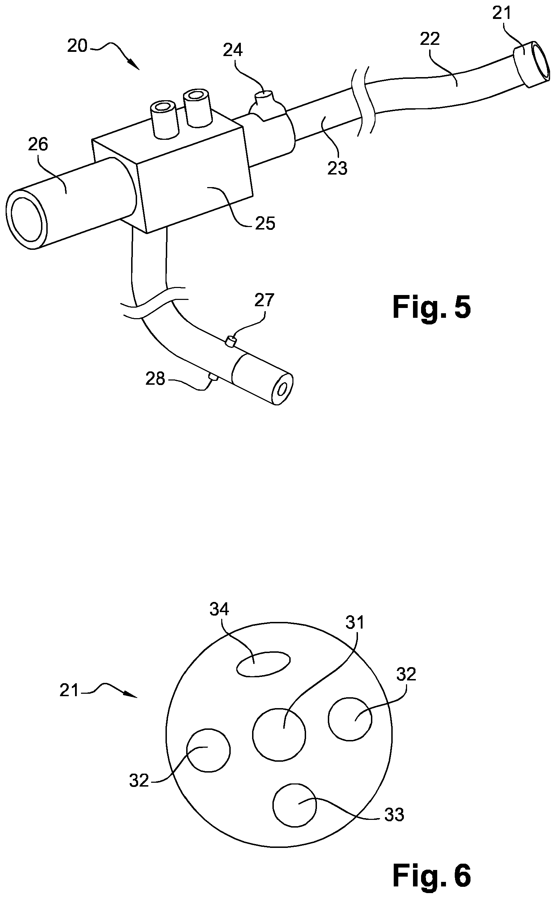

Le capuchon 1 comprend également un moyen de fixation 3, agencé de manière à permettre la fixation du capuchon 1 sur l'extrémité distale d'un endoscope, tel que celui illustré aux figures 5 et 6. Le moyen de fixation 3 est agencé de manière à se fixer à l'extrémité distale 21 de l'endoscope 20, de manière à ce que l'ouverture proximale 5 soit en vis-à-vis ou autour de l'extrémité distale 21 de l'endoscope 20.

Par exemple, tel qu'illustré, ce moyen de fixation est formé par un manchon 3 de rétention présentant une première extrémité 3a et une deuxième extrémité 3b.

Dans cet exemple, ce manchon 3 est destiné à être emmanché ou enfilé sur l'extrémité distale 21 de l'endoscope 20. Ainsi, la première extrémité 3a du manchon 3 est fixée sur l'extrémité distale 21 de l'endoscope 20. La deuxième extrémité 3b du manchon débouche dans l'ouverture proximale 5.

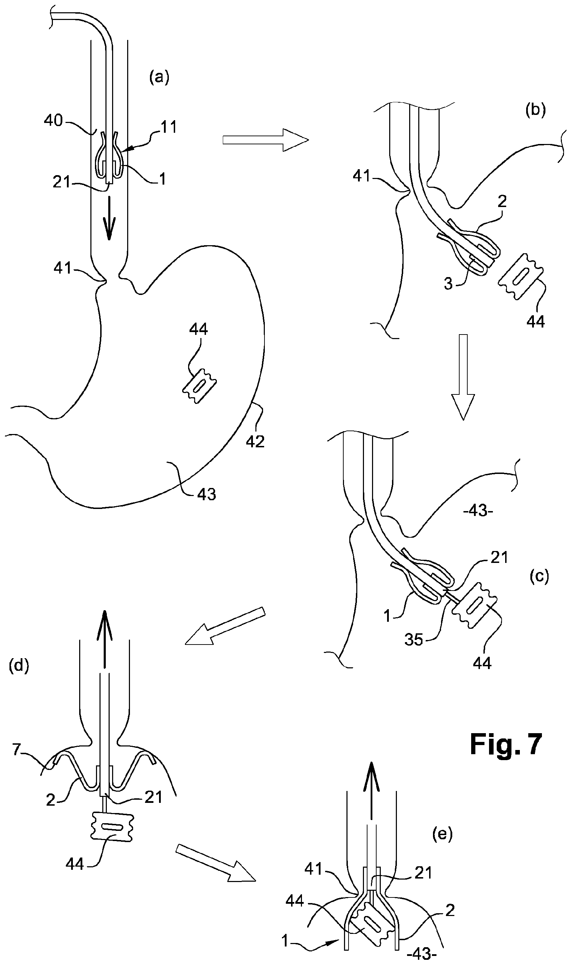

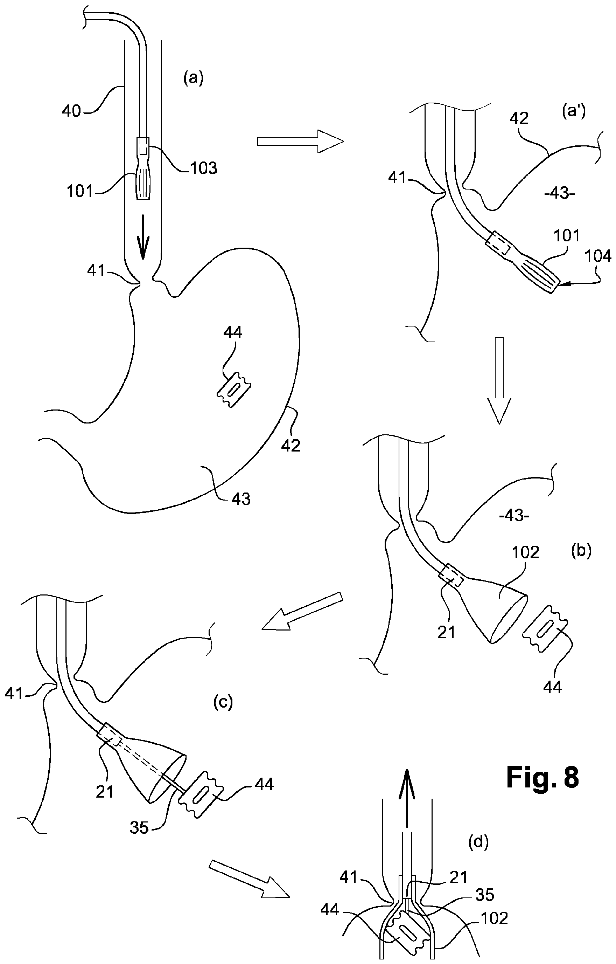

Comme on peut le voir sur les cinq étapes illustrées en figures 7 et 8, lorsque le manchon 3 est enfilé sur l'endoscope 20, l'extrémité distale 21 est à proximité de l'ouverture proximale 5 du capuchon 1. L'extrémité distale 21 comprenant un élément optique 21, tel qu'une lentille, reliée à un objectif 26, pour permettre à l'endoscopiste de voir en avant de cette extrémité distale 21, cet agencement du capuchon 1 permet de voir au-delà de l'ouverture proximale 5.

Selon la configuration de base de l'enveloppe 2, l'ouverture distale 4 est en vis-à-vis de l'ouverture proximale 5 et l'enveloppe présente une forme s'évasant depuis son ouverture proximale 5, ici jusqu'à l'ouverture distale 4. La visibilité au moyen de l'endoscope 20 est donc possible en dehors du capuchon 1.

Selon l'invention, et en particulier dans cet exemple, la paroi de l'enveloppe 2 est réalisée en une structure présentant une résistance suffisante pour résister à une perforation et/ou à une incision par un objet pointu et/ou tranchant. Cela permet donc d'aller rechercher un corps étranger dans la cavité d'un organisme, de le saisir et de l'enfermer dans l'enveloppe 2 avant de le retirer de la cavité, sans léser les tissus de cette cavité ou ceux situés entre cette cavité et un orifice de cet organisme. Ces utilisations seront détaillées plus loin.

Ici, l'enveloppe 2 a la forme d'une cloche et permet donc de recouvrir complètement un corps étranger, à l'intérieur d'un logement définit par cette enveloppe 2 en configuration de base. L'ouverture proximale 5 est agencée au sommet 6 de cette cloche, et l'ouverture distale 4 étant agencée à la base 7 de cette cloche.

L'enveloppe 2 peut présenter des dimensions de manière à ce que son logement comprenne un volume de 4 centimètres (cm) de diamètre et 5-6 cm de long. Ainsi, l'enveloppe 2, dont la longueur est supérieure dans ce cas à 5 cm, peut englober des objets de 5 cm de long et 4 cm de diamètre. L'ouverture distale 4 peut présenter un diamètre d'au moins 4 cm. Cela permet d'englober par exemple une aiguille, un morceau de verre ou une lame de rasoir. Ces dimensions sont adaptées à une extraction d'un corps étranger de l'estomac d'un humain. En cas d'usage vétérinaire, elles pourront être adaptées en fonction de la taille de l'animal, en particulier de la taille des voies naturelles de ce dernier.

Le manchon peut être sensiblement tubulaire et avoir un diamètre de 9 à 11 millimètres (mm). Cette taille n'est pas limitative et dépend de l'endoscope auquel le capuchon est destiné. Par exemple, un diamètre de 9 à 11 mm est adapté à un endoscope adulte dont le diamètre de l'extrémité distale est en général de 11 mm. Dans ce cas une fixation du manchon par enfilement est possible.

Dans le cas d'un endoscope pour nouveau-né, encore appelé neonatoscope, le diamètre peut être de 5,9 mm. Dans un tel cas, le manchon pourra être conçu avec un diamètre de 4 à 5,9 mm.

La paroi de l'enveloppe 2 peut être formée uniquement d'un ou plusieurs polymères. Il s'agit de polymères présentant une résistance suffisante pour résister à une perforation et/ou à une incision par un objet pointu et/ou tranchant.

Alternativement au paragraphe précédent, telle qu'illustrée en figure 4, l'enveloppe 2 comprend un renfort 8.

Dans cet exemple, le renfort 8 est agencé sur le tour de la paroi de l'enveloppe 2. Cela forme donc un renforcement autour du logement défini part l'enveloppe 2.

Le renfort 8 peut également, comme dans l'exemple illustré, être intégré dans la quasi-totalité, voire la totalité, de la paroi de l'enveloppe 2, ici, du sommet 6 à la base 7 de la forme en cloche de l'enveloppe.

Ce renfort est ici formé d'un ou de plusieurs fils entrelacés, formant ainsi une grille 8 souple.

Cette grille 8 est en fil(s) métallique(s). Cette grille métallique offre ainsi une bonne résistance à l'incision. L'enveloppe 2 est donc apte à entourer une lame de rasoir sans être traversée par cette dernière.

Le ou les fils formant la grille 8 peuvent être tels que ceux utilisés dans les endoprothèses tubulaires, encore appelées « stents », notamment avec le même maillage. De tels maillages sont décrits dans le document *"*Van Boeckel, P. G. A., Sijbring, A., Vleggaar, F. P. & Siersema, P. D. Systematic review: temporary stent placement for benign rupture or anastomotic leak of the oesophagus. Aliment. Pharmacol. Ther. 33, 1292-1301 (2011*)."* **[8].**

Le maillage peut comprendre des mailles d'environ 1 mm² de côté.

Selon l'invention, il est également possible de choisir un maillage avec des mailles dont les côtés sont inférieurs à 1 mm² pour diminuer le risque de perforation, en particulier par des aiguilles. Dans ce cas, on peut choisir le maillage de manière à ce que l'enveloppe 2 ait une plasticité suffisante pour se déformer lors du passage de l'orifice de la cavité, notamment le cardia dans le cas où le capuchon 1 est utilisé sur un endoscope pour le retrait d'un corps étranger dans l'estomac.

Le matériau de ce ou ces fils peut être choisi parmi : un acier inoxydable, un nitinol, le tantale, des alliages de cobalt et de chrome, notamment MP35N ou MP20N, le platine, le titane.

Notamment, ces fils peuvent être d'un alliage métallique à mémoire de forme, tel qu'un alliage de nickel et de titane, dans des proportions molaires quasi-identiques, voir identiques. Par exemple, ce peut être un alliage de nickel et de titane couramment appelé nitinol.

Par exemple, le ou les fils de la grille 8 peuvent être formés d'un alliage de Nickel et de Titane contenant 50 à 51% de Nickel, en pourcentage molaire, soit 55 à 56 % en pourcentage massique. Cet alliage est un matériau à mémoire de forme.

En figure 4, seule la grille 8 est représentée en traits pleins pour permettre de la visualiser. La surface extérieure du capuchon 1, donc également de l'enveloppe 2, est représentée par des pointillés.

En effet, dans cet exemple, la grille 8 est recouverte par deux couches de recouvrement de part et d'autre de cette grille 8. Ces couches sont formées d'un polymère de recouvrement, dans cet exemple identique pour chacune des couches.

La figure 1 peut illustrer le capuchon 1 de la figure 4. Dans ce cas, la grille 2 n'est donc pas visible sur cette figure 1, puisqu'elle est recouverte à la fois à l'intérieur du logement défini par l'enveloppe 2 par une couche de recouvrement interne, et à l'extérieur de ce logement par une couche de recouvrement externe.

Dans cet exemple, les deux couches de recouvrement de part et d'autre de la grille 8 forment une seule pièce en polymère comprenant une âme formée par la grille 8.

Au niveau de la base 7 de l'enveloppe, le bord de la paroi peut être arrondi afin de moins irriter les tissus en cas de frottement. Par exemple, la couche de recouvrement externe peut être repliée par-dessus la grille, couvrant le bord de celle-ci, en formant ainsi un ourlet.

Par ailleurs, il est possible, comme dans cet exemple, de former ces couches en une seule pièce avec le manchon 3 de rétention. Ainsi, la paroi de l'enveloppe 2 et ce manchon 3 sont formés en une seule pièce, en d'autres termes sont venus de matière en une seule pièce. Par exemple, le capuchon 1 peut être obtenu par surmoulage du ou des polymères autour de la grille 8, celle-ci étant placée dans l'empreinte du moule du capuchon, le polymère étant ensuite injecté.

Dans cet exemple, c'est donc le même polymère ou mélange de polymères qui forment les couches de recouvrement et le manchon 3.

Cependant selon une variante non représentée, le manchon et les couches de recouvrement peuvent être formés de polymères distincts, à savoir le ou les polymères du manchon, le ou les polymères de recouvrement interne de la grille, le ou les polymères de recouvrement externe de la grille. Les termes « interne » et « externe » sont définis par rapport aux positions des couches de recouvrement dans la configuration de base.

Différents types de polymères peuvent être utilisés, notamment ceux utilisés dans les endoprothèses tubulaires, notamment ceux décrits dans « Van Boeckel, P. G. A., Sijbring, A., Vleggaar, F. P. & Siersema, P. D. Systematic review: temporary stent placement for benign rupture or anastomotic leak of the oesophagus. Aliment. Pharmacol. Ther. 33, 1292-1301 (2011) » **[8].**

Ces polymères peuvent être choisis parmi : la silicone, le polyuréthane, le polytétrafluoroéthylène (PTFE).

Le ou les polymères du manchon 3 peuvent être poreux pour améliorer l'adhérence à l'extrémité de l'endoscope.

Le ou les polymères de recouvrement interne et/ou externe peuvent également être un ou des polymères résistant à la perforation. Cela permet de renforcer davantage l'enveloppe 2. Cela permet également de diminuer le risque qu'une aiguille que l'on extrait de l'organisme, passe au travers des mailles de la grille 8 lors du retrait.

Cette grille 8 et les polymères, et par conséquent la paroi du capuchon 1 présentent une certaine rigidité et une certaine souplesse.

Dans l'exemple illustré en figures 1, 2, et 4, la grille ne débute qu'au niveau du sommet 6 de l'enveloppe 2 et est donc exclusivement dans l'enveloppe 2, le manchon 3 en étant dépourvu. Ainsi, la zone adjacente à la grille 8 au niveau de la deuxième extrémité 3b du manchon va être plus souple que la paroi de l'enveloppe 2 et former de ce fait une articulation. Cette articulation permet de retourner l'enveloppe 2 sur elle-même autour du manchon 3, qui se retrouve alors enfermé par cette paroi, comme illustré en figure 2.

Lors de ce retournement, l'enveloppe 2 passe de la configuration de base de la figure 1 à une configuration repliée, illustrée en figure 2. Ainsi, la face interne 11 de la paroi du logement en configuration de base passe à l'extérieur en configuration repliée, et la face externe 12 de la paroi du logement en configuration de base passe à l'intérieur en configuration repliée.

Le capuchon 1 peut à nouveau repasser de la configuration repliée à la configuration de base par déroulement dans l'autre sens, la configuration de base correspondant à une configuration dépliée.

Selon un autre exemple illustrée en figure 3, le capuchon 101 en configuration de base, ou configuration dépliée, est identique à celui de la figure 1 mais présente une configuration repliée, dite configuration compactée, distincte de la configuration repliée de l'exemple précédent.

Ici, l'enveloppe 102 en configuration compactée présente des portions de repli 109 repliées sur elles-mêmes, selon des plis s'étendant longitudinalement de l'ouverture distale 104 vers l'ouverture proximale 105 de l'enveloppe 102, soit du sommet 106 à la base 107 de cette dernière.

Cela permet de réduire l'évasement et donc le diamètre de l'enveloppe 102, pour permettre un passage plus facile dans la cavité.

Selon l'exemple illustré, les portions de repli sont maintenues repliées par un fil ou câble 113 agencé autour de l'enveloppe 102 au niveau de la base 107 de l'enveloppe. Il peut par exemple s'agir d'un câble en polypropylène.

Ce câble 113 peut notamment passer au travers de trous traversant la paroi de l'enveloppe 102 et passer de manière alternative d'un côté et de l'autre de cette paroi à la manière d'un lacet.

Selon une variante non représentée, ces trous peuvent ne pas traverser la paroi de part en part, mais le câble peut être uniquement piqué, comme une couture dans le matériau du ou des polymères de recouvrement à l'intérieur du logement défini par l'enveloppe.

Ce câble 113 se prolonge à l'intérieur du logement en direction du manchon 103, notamment jusqu'à ce dernier. Cela permet une fois le capuchon 101 monté sur l'endoscope, d'actionner le câble 113 par un actionneur passant par un canal opérateur 33 de l'endoscope 20 pour relâcher l'étreinte du câble 113, l'enveloppe 102 du fait de la souplesse du ou des polymères revenant alors à sa configuration de base, comme celle de la figure 1.

Alternativement ou en complément, le capuchon 101 peut comprendre une grille comme la grille 8 de l'exemple précédent, celle-ci étant en matériau à mémoire de forme dont la température de fin de passage en phase austénitique est comprise entre 34 et 42 degrés, notamment entre 36 et 40 degrés.

Cette grille présente comme forme mémorisée une forme correspondant à celle de la configuration de base, par exemple comme celle de la grille 8 de la figure 4.

Il est possible de former les portions repliées 109 par déformation de la grille, donc torsion de ses fils, avant introduction de l'endoscope dans l'organisme. Le matériau à mémoire de forme est alors dans sa forme martensitique.

Une fois le capuchon 101 introduit dans la cavité, le matériau à mémoire de forme est chauffé et passe en phase austénitique. La grille retrouve alors la forme mémorisée, et donc l'enveloppe 102 sa configuration de base.

Dans un tel cas, le capuchon 101 peut donc être dépourvu de câble 113. Aucune action n'est alors nécessaire de la part de l'endoscopiste pour que le capuchon 101 s'ouvre.

Pour obtenir un capuchon 1 ou 101 avec un renfort, tel que la grille 8, plusieurs procédés de fabrication sont possibles.

Selon un procédé de fabrication, le matériau de la grille 8 est en métal, notamment choisi parmi des matériaux à mémoire de forme dont la température de fin de passage en phase austénitique est comprise entre 34 et 42 degrés, notamment un nitinol, tel qu'évoqué précédemment.

Un procédé selon l'invention comprend :
- une étape de réalisation et/ou de conformation de la grille 8 en forme de manche dont la forme correspond à au moins une partie de la forme de configuration de base,
- une étape de recouvrement de la grille 8 des deux côtés par un polymère de recouvrement, notamment par surmoulage d'un polymère sur la grille dans un moule dont l'empreinte correspond à la forme du manchon.

Lors de l'étape de conformation de la grille 8, il est possible de faire mémoriser à celle-ci la forme correspondant à la configuration de base. Pour cela, la grille 8, en particulier son ou ses fils, peut être contrainte mécaniquement par exemple par serrage entre une forme et une contreforme, ou par tissage des fils. Cette contrainte est réalisée à une température suffisante pour que cette forme devienne la forme mémorisée par la grille 8, par exemple 500°C.

L'étape de conformation est ensuite suivie d'une étape de refroidissement de la grille 8 en dessous de la température de fin de passage en phase martensitique du matériau du ou des fils de la grille 8. La grille 8 est alors en phase martensitique dans la forme correspondant à la configuration de base.

L'étape de recouvrement est ensuite mise en œuvre.

Dans le cas où l'on souhaite réaliser un capuchon 101 prêt à l'emploi selon la configuration compactée, telle qu'illustrée en figure 3, on met ensuite en œuvre une étape de compactage de la grille, par formation des portions repliées 109, tout en conservant l'ouverture distale 104 en vis à vis de l'ouverture proximale 105. Cette étape de compactage est réalisée en dessous de la température de fin de passage en phase martensitique du matériau de la grille. L'enveloppe 102 conserve ainsi sa forme compactée, la grille étant tordue.

Concernant l'étape de recouvrement, d'autres techniques que le surmoulage peuvent être employées.

En particulier, la grille 8 peut être imprégnée par le ou les polymères par trempage dans le matériau qui polymérise ensuite autour du ou des fils de la grille. Le manchon 3, 103 peut être formé séparément et ensuite collé à l'enveloppe 2, 102. Il est également possible de former entièrement le capuchon avec une grille qui s'étend également dans tout le manchon. Une simple imprégnation est alors suffisante.

Le recouvrement peut également être réalisé par pulvérisation (spray).

On peut notamment utiliser les techniques de recouvrement divulguées dans la demande US 2007/288088 A1.

Il est également possible de réaliser une couche interne et une couche externe en matériau(x) polymère(s) à la forme adaptée et de les coller ou de les souder de part et d'autre de la grille.

Le capuchon 1, 101, selon l'invention, notamment selon les exemples décrits, est particulièrement utile pour une utilisation du capuchon dans un procédé d'extraction d'un corps étranger d'une cavité interne d'un organisme humain ou animal au moyen d'un endoscope.

Un exemple d'endoscope 20 est illustré en figures 5 et 6. Celui-ci comprend un tube présentant d'un côté une section flexible 23, reliée à un module de contrôle, et de l'autre une section terminale à courbure contrôlable 22. Cette dernière se termine par une extrémité distale 21 destinée à être introduite dans un organisme, en particulier par une voie naturelle. La courbure et l'orientation de la section terminale 22 sont contrôlées depuis le module de contrôle 25.

Le tube comprend des moyens optiques permettant d'acheminer les images en avant de l'extrémité distale 21, depuis une lentille optique 31 de l'extrémité distale 21, jusqu'à un objectif 26, situé au niveau du module de contrôle 25. L'opérateur, à savoir l'endoscopiste, peut ainsi voir par l'objectif 26 en avant de l'endoscope 20.

L'extrémité distale 21 comprend également deux dispositifs d'éclairage 32 pour éclairer devant l'endoscope 20.

Le tube comprend un canal opérateur 33, présentant une entrée 24 au niveau du module de contrôle 25 et débouchant au niveau de l'extrémité distale 21 par une sortie du canal opérateur. Ce canal opérateur 33 peut permettre le passage d'un bras de préhension, non représenté en figures 5 et 6, et de moyens d'actionnement. Le bras peut alors être actionné par l'endoscopiste et sortir en avant de l'extrémité distale 21.

De façon schématique, sont représentées un groupe d'entrées 27 et de sorties 28, permettant d'injecter un gaz, notamment de l'air, et d'injecter de l'eau, et également de les aspirer, via actionnement du module de contrôle 25. L'extrémité distale 21 présente une buse 34 permettant un envoi de l'eau ou de l'air, notamment pour nettoyer la lentille 31.

Comme expliqué précédemment, selon l'invention le manchon 3 peut être conçu avec un diamètre inférieur ou égal à celui de l'endoscope qui en est équipé. Dans un tel cas, par exemple tel qu'illustré précédemment, le manchon 3 est enfilé sur l'extrémité distale 21 de l'endoscope 20, son élasticité permettant une adaptation et une rétention du capuchon 1 sur l'endoscope.

Cependant lorsque des endoscopes sont très différents, il est possible de compléter la fixation par un élastique. Par exemple, en prenant un capuchon avec un manchon 3 de 9 mm de diamètre, la fixation pourra se faire sur un endoscope pour adulte sans utiliser d'élastique. Ce même capuchon 1 peut être utilisé sur un neonatoscope de 5,9 mm, en enfilant le manchon 3 sur l'extrémité distale du neonatoscope, puis en passant un élastique autour du manchon 3.

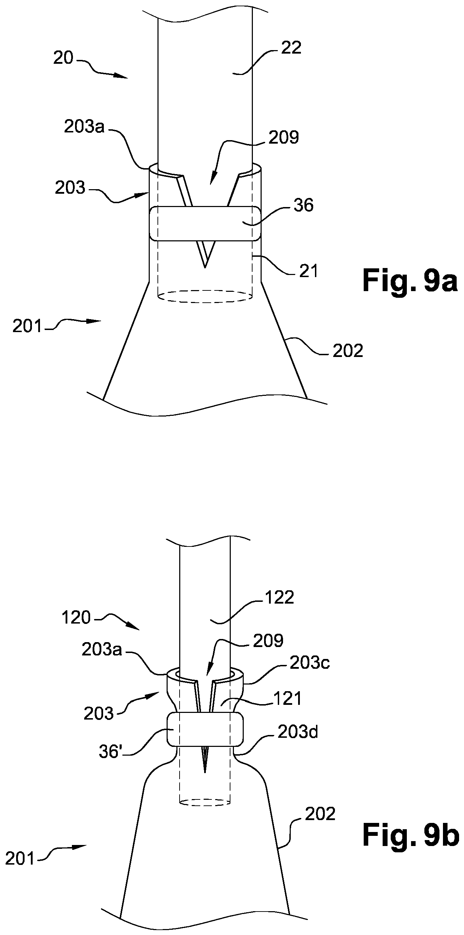

Selon l'invention, notamment selon l'exemple illustré aux figures 9a et 9b, le manchon 203 du capuchon 201 peut comprendre une fente longitudinale 209 évasée vers la première extrémité 203a du manchon 203.

Lorsque, comme en figure 9a, le manchon 203 est monté sur cet endoscope 20, il conserve une forme environ cylindrique autour de l'extrémité distale 21. Un élastique 36 est ensuite passé autour du manchon pour maintenir ce dernier.

Lorsque, comme en figure 9b, le manchon 203 est monté sur un neonatoscope 120, les bords de la fente 209 sont rapprochés et maintenus ainsi par l'ajout d'un élastique 36' plus petit ou plus serré. L'élastique 36' sépare ainsi le manchon 203 en une partie aval 203d, entre l'enveloppe 202 et l'élastique 36', et une partie amont de l'autre côté de l'élastique 36'. Ainsi grâce à cette fente, les parois du manchon 203, notamment dans la partie amont 203c sont rapprochées du tube 122 du neonatoscope 120. Cette partie amont 203c est ainsi moins évasée qu'en l'absence de la fente 209. Cette dernière améliore ainsi l'adaptabilité du capuchon 201 à un endoscope 120 à extrémité distale 121 de diamètre plus faible. Cela facilite également le retournement de l'enveloppe 202 sur elle-même autour du manchon 203.

Dans ces différentes variantes, la paroi formant l'enveloppe est continue sur toute sa périphérie.

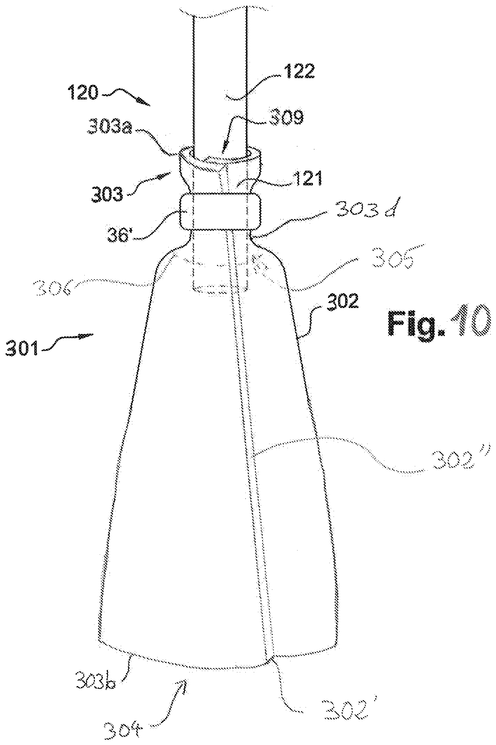

Selon une variante, telle qu'illustrée en figure 10, la paroi 302' formant l'enveloppe 302 est discontinue et fendue sur toute sa longueur, soit d'une extrémité proximale 303a à une extrémité distale 303b. L'enveloppe 302 est obtenue par enroulement de la paroi 302' sur elle-même dans le sens de la longueur, de manière à fermer l'enveloppe 302 entre l'extrémité proximale 303a, destinée à être autour de l'endoscope, ici le neonatoscope 120, et l'extrémité distale 303b, qui forme l'ouverture distale 304.

Dans ce cas, le capuchon 301 peut être monté autour de l'endoscope 120 par enroulement de la paroi 302' autour du tube 122 de l'endoscope, jusqu'à ce que la paroi 302' se chevauche elle-même au moins partiellement selon sa longueur. On a ainsi un logement formé en avant de l'endoscope 120. Dans l'exemple illustré, la zone de chevauchement 309 de la paroi 302' sur elle-même est limitée mais pourrait être plus ou moins importante.

L'une 302" des tranches longitudinales de la paroi 302' se retrouve ainsi visible à l'extérieur de l'enveloppe 302.

Ensuite, une attache, tel qu'un élastique 36', peut être passée autour de la paroi 302' enroulée et de l'extrémité distale 121 de l'endoscope de manière à fixer le capuchon 302 à l'endoscope 120. Le capuchon 302 présente ainsi le manchon 303 d'un côté de l'élastique 36', et l'enveloppe 302 de l'autre côté de cet élastique 36'. Autrement dit, le capuchon 301 est formé par une cape, dont l'enroulement et l'attache autour de l'endoscope 120 forme le logement entre l'ouverture proximale 305 et l'ouverture distale 304 de l'enveloppe 302.

La cape peut comprendre un renfort tel que décrit précédemment, notamment un maillage de fil (non représenté), notamment des fils en matériau à mémoire de forme. Dans ce cas, le maillage peut s'étendre sur une portion aval de la cape, dite portion renforcée. Cette portion renforcée forme l'essentiel de l'enveloppe 302.

Dans l'exemple illustré, la portion renforcée s'étant entre une limite proximale 306 et l'ouverture distale 304 de l'enveloppe 302.

L'élastique 36' peut être fixé à l'écart de cette limite proximale 306 de manière à laisser une portion aval 303d du manchon 303 juste entre l'élastique 36' et cette limite proximale 306. Cette portion aval 303d du manchon 303 étant dépourvue de renfort, ici de maillage, elle forme une articulation de l'enveloppe, qui lui permet d'être retournée sur elle-même et par-dessus le manchon 303, avant l'insertion de l'endoscope 120 dans le patient.

La réalisation de cette cape peut être mise en œuvre selon le procédé de fabrication précédemment décrit, hormis que l'étape de réalisation et/ou de conformation du renfort est réalisée différemment. Dans un tel cas en effet, le renfort, notamment une grille, est réalisé en forme de cape, et non de manche, par exemple une bande trapézoïdale.

La figure 7 illustre un exemple d'utilisation du capuchon 1 des figures 1, 2 et 4 dans un procédé d'extraction d'une lame de rasoir 44, ingérée volontairement et logée à l'intérieur de la cavité 43 définie par l'estomac 42 d'un être humain.

Le manchon 3 est d'abord enfilé sur l'extrémité distale 21 de l'endoscope 20, et éventuellement ajusté en profondeur. Le manchon peut comprendre un bourrelet, non représenté, venant se loger en arrière de l'extrémité distale 21 pour prévenir tout risque de perte du capuchon 1 dans l'estomac 42. La fixation peut être complétée par un élastique.

Ensuite, l'enveloppe 2 est retournée en arrière, par-dessus et autour du manchon 3.

L'endoscopiste met ensuite en œuvre une étape de pénétration (a) de l'organisme, via la bouche de la personne. Le tube de l'endoscope est ensuite introduit jusqu'à l'estomac en passant par l'œsophage 40.

L'enveloppe 2 étant retournée, sa surface interne 11 est à l'extérieur et peut être amenée à frotter contre les parois de l'œsophage 40 ou du cardia 41 avant l'entrée dans l'estomac 42. Cependant, la couche de recouvrement interne permet de protéger ces parois de toute irritation en particulier par la grille 8.

Ensuite, l'endoscopiste procède à une étape de recherche (b) de la lame de rasoir 44. L'enveloppe 2 étant retournée, elle laisse un champ de vision très large.

Une fois la lame 44 repérée, l'endoscopiste procède à une étape de saisie (c) de cette lame 44 par le bras de préhension 35 de l'endoscope 20, qui sort du canal opérateur 33.

Le bras 35 est ensuite rétracté et le tube de l'endoscope est peu à peu retiré. A un moment donné, l'extrémité distale 21 s'approche du cardia 41 et la base 7 de l'enveloppe entre en contact avec les bords du cardia 41. La force exercée par le mouvement de retrait de l'extrémité distale 21 de l'estomac 42 pousse cette base 7 vers l'avant, et entraine le retournement progressif de l'enveloppe 2. Au fur et à mesure du retrait de la cavité 43, l'enveloppe 2 se déroule et enveloppe ainsi la lame de rasoir 44, avant que l'enveloppe 2 ne sorte de la cavité 43 stomacale. C'est donc le passage de cardia 41 qui réalise l'étape d'enveloppement (d) de la lame.

Il est alors possible de débuter l'étape de retrait (e) du tube de l'endoscope 20 de l'organisme. Comme on peut le voir, l'enveloppe 2 renferme complètement la lame de rasoir 44 et permet le passage du cardia 41 sans léser celui-ci. Egalement, cela protègera la paroi de l'œsophage 40.

Par sa structure, l'enveloppe 2, malgré les frottements, ne sera pas transpercée par la lame de rasoir 44.

La figure 8 illustre un exemple d'utilisation du capuchon 101 de la figure 3 dans un procédé d'extraction d'une lame de rasoir 44, ingérée volontairement et logée à l'intérieur de la cavité 43 définie par l'estomac 42 d'un être humain.

Le manchon 103 est d'abord enfilé sur l'extrémité distale 21 de l'endoscope 20, et éventuellement ajusté en profondeur de la même manière que pour l'utilisation en figure 7.

Ensuite, l'enveloppe est repliée par une étape de compactage, sauf si le capuchon 101 est livré déjà compacté. Cette étape de compactage est identique à celle déjà décrite précédemment en référence à la figure 3.

L'endoscopique met ensuite en œuvre une étape de pénétration (a) de l'organisme jusqu'à la cavité stomacale 43, comme dans l'exemple précédent, via la bouche de la personne, puis l'œsophage 40.

Le capuchon 101 comprend ici une grille en un matériau à mémoire de forme comme décrit précédemment.

Du fait de sa forme compactée, le capuchon 101 frotte peu sur les parois de l'organisme. Par ailleurs, la couche de polymère de recouvrement externe permet de protéger les tissus de la grille du capuchon.

Comme les plis sont longitudinaux, l'ouverture distale 104 reste devant l'ouverture proximale 105 et permet un premier guidage.

Une fois dans l'estomac 42, soit l'étape (a') en figure 8, la chaleur déclenche le dépliement et le déploiement de l'enveloppe 102. La forme en cloche de l'enveloppe 102 offre un bon angle de vision par l'extrémité distale 21 et l'endoscopiste peut procéder à une étape de recherche (b) de la lame de rasoir 44.

Une fois la lame 44 repérée, l'endoscopiste procède à une étape de saisie (c) de cette lame 44 par bras de préhension 35 de l'endoscope 20 qui sort du canal opérateur 33.

Le bras 35 est ensuite rétracté amenant la lame de rasoir 44 à l'intérieur de l'enveloppe 102.

Il est alors possible de débuter l'étape de retrait (d) du tube de l'endoscope de l'organisme. Dans cet exemple également, l'enveloppe 102 renferme complètement la lame de rasoir 44 et permet le passage du cardia 41 et de l'œsophage 40 sans léser ceux-ci.

Bien que ces exemples aient été décrits dans le cadre de l'extraction d'un corps étranger de l'estomac d'un être humain, cela pourrait s'appliquer pour un usage vétérinaire, par exemple dans le cas de bouts de verre contenus dans un aliment ingéré par l'animal.

Egalement, le corps étranger peut être extrait d'une autre cavité que l'estomac. La pénétration initiale peut également ne pas être réalisée par voie naturelle mais après incision pour accéder à la cavité, afin de ne pas léser davantage les tissus incisés.

Un avantage supplémentaire du capuchon est qu'il est simple à produire, notamment à partir de matériaux employés dans le domaine médical. Il peut donc être produit pour un faible coût et être ainsi plus facilement utilisé pour un usage unique.

### Liste des références

[1] Hesham A-Kader, H. Foreign body ingestion: children like to put objects in their mouth. World J. Pediatr. WJP 6, 301-310 (2010).
**[2]** Olives, J.-P., Bellaïche, M. & Michaud, L. [Digestive foreign bodies in children]. Arch. Pédiatrie Organe Off. Sociéte Fr. Pédiatrie 16, 962-964 (2009).
[3] Chauvin, A., Viala, J., Marteau, P., Hermann, P. & Dray, X. Management and endoscopic techniques for digestive foreign body and food bolus impaction. Dig. Liver Dis. Off. J. Ital. Soc. Gastroenterol. Ital. Assoc. Study Liver 45, 529-542 (2013).
**[4]** Goh, B. K. P. et al. Perforation of the Gastrointestinal Tract Secondary to Ingestion of Foreign Bodies. World J. Surg. 30, 372-377 (2006).
**[5]** Ikenberry, S. O. et al. Management of ingested foreign bodies and food impactions. Gastrointest. Endosc. 73, 1085-1091 (2011).
**[6]** Velitchkov, N. G., Grigorov, G. I., Losanoff, J. E. & Kjossev, K. T. Ingested foreign bodies of the gastrointestinal tract: retrospective analysis of 542 cases. World J. Surg. 20, 1001-1005 (1996).
[7] Michaud, L., Bellaïche, M. & Olives, J.-P. Ingestion de corps étrangers chez l'enfant. Recommandations du Groupe francophone d'hépatologie, gastroentérologie et nutrition pédiatriques. Arch. Pédiatrie 16, 54-61 (2009).
[8] Van Boeckel, P. G. A., Sijbring, A., Vleggaar, F. P. & Siersema, P. D. Systematic review: temporary stent placement for benign rupture or anastomotic leak of the oesophagus. Aliment. Pharmacol. Ther. 33, 1292-1301 (2011).

## Revendications

1. Capuchon (1 ; 101 ; 201 ; 301) d'endoscope comprenant :
- une enveloppe (2; 102; 202; 302) de protection formée par une paroi repliée sur elle-même de manière à ce que ladite enveloppe comprenne une ouverture proximale (5 ; 105 ; 305) et une ouverture distale (4 ; 104 ; 304) en vis-à-vis de l'ouverture proximale et présente une forme s'évasant depuis son ouverture proximale vers son ouverture distale, selon une configuration de base, dans laquelle ladite enveloppe (2 ; 102 ; 202) définit un logement,
- un moyen de fixation (3; 103; 203; 303) agencé de manière à permettre la fixation du capuchon sur l'extrémité distale d'un endoscope (20, 120) avec ladite ouverture proximale (5 ; 105; 305) en vis-à-vis ou autour de l'extrémité distale (21, 121) de l'endoscope,
**caractérisé en ce que** la paroi de ladite enveloppe est réalisée en une structure présentant une résistance suffisante pour résister à une perforation et/ou à une incision par un objet pointu et/ou tranchant, la paroi de ladite enveloppe comprenant un renfort (8) agencé de manière à entourer ledit logement, le renfort étant formé d'un ou plusieurs fils.

2. Capuchon (1 ; 101 ; 201 ; 301) selon la revendication 1, dans lequel ladite enveloppe (2 ; 102; 202; 302) forme une cloche, l'ouverture proximale (5 ; 105 ; 305) étant agencée au sommet (6 ; 106) de cette cloche, et l'ouverture distale (4 ; 104 ; 304) étant agencée à la base (7 ; 107) de cette cloche.

3. Capuchon (1 ; 101 ; 201 ; 301) selon la revendication 1 ou 2, dans lequel le renfort peut être formé par un ou des fils constitués d'un matériau à mémoire de forme.

4. Capuchon (1 ; 101 ; 201 ; 301) selon l'une quelconque des revendications précédentes, dans lequel le ou les fils forment un maillage.

5. Capuchon (1 ; 101 ; 201 ; 301) selon l'une quelconque des revendications précédentes, dans lequel ladite enveloppe (2 ; 102; 202 ; 302) comprend au moins une couche composée d'au moins un polymère de recouvrement externe recouvrant le renfort à l'extérieur dudit logement.

6. Capuchon (1 ; 101 ; 201 ; 301) selon la revendication 5, dans lequel ladite enveloppe (2; 102; 202; 302) comprend une autre couche composée d'au moins un polymère de recouvrement interne recouvrant le renfort à l'intérieur dudit logement.

7. Capuchon (1 ; 101 ; 201 ; 301) selon l'une quelconque des revendications précédentes, dans lequel la paroi de ladite enveloppe (2 ; 102 ; 202) est formée uniquement d'un ou plusieurs polymères.

8. Capuchon (1 ; 101 ; 201 ; 301) selon l'une quelconque des revendications précédentes, dans lequel le moyen de fixation comprend un manchon de rétention présentant une première extrémité destinée à se fixer sur l'extrémité distale de l'endoscope, le manchon de rétention présentant une deuxième extrémité fixée sur ladite enveloppe de manière à ce que le manchon débouche dans ladite ouverture proximale.

9. Capuchon (1 ; 101 ; 201 ; 301) selon la revendication 8, dans lequel le manchon (3 ; 103 ; 203 ; 303) et la paroi de ladite enveloppe sont venus de matière en une seule pièce.

10. Capuchon (1 ; 101 ; 201 ; 301) selon la revendication 8 ou 9, dans lequel le manchon (3 ; 103 ; 203 ; 303) est constitué d'un matériau composé d'un ou plusieurs polymères, dits polymères du manchon.

11. Capuchon (1 ; 101 ; 201 ; 301) selon la revendication 10, dans lequel le matériau constituant le manchon (3; 103; 203 ; 303) est élastique et/ou poreux.

12. Capuchon (1 ; 101 ; 201 ; 301) selon l'une quelconque des revendications 8 à 11, dans lequel le manchon (3; 103; 203; 303) présente une surface interne comprenant au moins un creux et/ou au moins un relief.

13. Capuchon (1 ; 201 ; 301) selon l'une quelconque des revendications précédentes, dans lequel la paroi présente une souplesse permettant à l'enveloppe (2 ; 202 ; 302) d'être mise dans une configuration repliée, dans laquelle l'enveloppe est retournée sur elle-même autour du moyen de fixation, et de passer par déroulement de cette configuration à une configuration dépliée, correspondant à la configuration de base et dans laquelle ladite enveloppe est retournée dans l'autre sens de sorte qu'elle est agencée à côté du moyen de fixation (3 ; 203 ; 303).

14. Capuchon (1 ; 201 ; 301) selon la revendication 13, dans lequel le renfort ne débute qu'au niveau du sommet (6) de l'enveloppe (2 ; 202 ; 302) et est exclusivement dans l'enveloppe, le moyen de fixation (3 ; 203 ; 303) en étant dépourvu, la zone adjacente (303d) au renfort étant ainsi plus souple que la paroi de l'enveloppe et formant de ce fait une articulation autour duquel l'enveloppe est apte à être retournée sur elle-même dans sa configuration repliée.

15. Capuchon (101) selon l'une quelconque des revendications 1 à 12, dans lequel l'enveloppe (102) est agencée de manière à pouvoir passer, par déformation, d'une configuration compactée à une configuration déployée, correspondant à la configuration de base, l'enveloppe présentant dans sa configuration compactée un évasement réduit de l'enveloppe par rapport à la configuration de base ou étant dépourvue d'évasement, tout en conservant l'ouverture distale (104) en vis à vis de l'ouverture proximale (105).

16. Capuchon (301) selon l'une quelconque des revendications précédentes, dans lequel la paroi (302') formant l'enveloppe (302) est discontinue et fendue sur toute sa longueur, d'une extrémité proximale (303a) à une extrémité distale (303b), de manière à ce que l'enveloppe (302) puisse être obtenue par enroulement de la paroi (302') sur elle-même dans le sens de la longueur, de manière à fermer l'enveloppe entre l'extrémité proximale (303a), destinée à être autour de l'endoscope, et l'extrémité distale (303b), qui forme ainsi les bords de l'ouverture distale (304).

17. Procédé de fabrication d'un capuchon (1; 101; 201) d'endoscope (20, 120), ledit capuchon étant selon l'une quelconque des revendications 1 à 16, ledit capuchon comprenant une enveloppe (2 ; 102 ; 202) comprenant une ouverture proximale (5 ; 105) et une ouverture distale (4; 104) en vis-à-vis de l'ouverture proximale et présentant une forme d'une configuration de base, cette forme s'évasant depuis son ouverture proximale vers son ouverture distale, ledit procédé de fabrication, comprenant:
- une étape de réalisation et/ou de conformation d'un renfort (8) soit en forme de manche dont la forme correspond à au moins une partie de la forme de configuration de base, soit en forme de cape,
- une étape de recouvrement du renfort par une couche composée d'au moins un polymère de recouvrement sur au moins l'une des faces du renfort,
le matériau du renfort et/ou le ou les polymères de recouvrement étant résistant(s) à une perforation et/ou à une incision par un objet pointu et/ou tranchant, ledit procédé de fabrication.

## Patentansprüche

1. Endoskopkappe (1; 101; 201; 301), umfassend:
- eine Schutzhülle (2; 102; 202; 302), die von einer Wand gebildet wird, welche so um sich selbst gebogen ist, dass die Hülle in einer Grundkonfiguration, in der die Hülle (2; 102; 202) eine Aufnahme definiert, eine proximale Öffnung (5; 105; 305) und eine der proximalen Öffnung gegenüberliegende distale Öffnung (4; 104; 304) umfasst und eine Form aufweist, die sich von ihrer proximalen Öffnung zu ihrer distalen Öffnung hin aufweitet,
- ein Befestigungsmittel (3; 103; 203; 303), das so angeordnet ist, dass es die Befestigung der Kappe am distalen Ende eines Endoskops (20, 120) mit der proximalen Öffnung (5; 105; 305) dem distalen Ende (21, 121) des Endoskops gegenüberliegend oder um dasselbe herum ermöglicht,
**dadurch gekennzeichnet, dass** die Wand der Hülle aus einer Struktur ausgeführt ist, die eine ausreichende Festigkeit aufweist, um einer Perforation und/oder einem Einschnitt durch ein spitzes und/oder scharfkantiges Objekt standzuhalten, wobei die Wand der Hülle eine Verstärkung (8) umfasst, die so angeordnet ist, dass sie die Aufnahme umgibt, wobei die Verstärkung von einem oder mehreren Drähten gebildet wird.

2. Kappe (1; 101; 201; 301) nach Anspruch 1, wobei die Hülle (2; 102; 202; 302) eine Glocke bildet, wobei die proximale Öffnung (5; 105; 305) an der Spitze (6; 106) dieser Glocke angeordnet ist, und wobei die distale Öffnung (4; 104; 304) an der Basis (7; 107) dieser Glocke angeordnet ist.

3. Kappe (1; 101; 201; 301) nach Anspruch 1 oder 2, wobei die Verstärkung von einem oder mehreren Drähten gebildet werden kann, die aus einem Formgedächtnismaterial bestehen.

4. Kappe (1; 101; 201; 301) nach einem der vorstehenden Ansprüche, wobei der oder die Drähte ein Geflecht bilden.

5. Kappe (1; 101; 201; 301) nach einem der vorstehenden Ansprüche, wobei die Hülle (2; 102; 202; 302) mindestens eine Schicht umfasst, die aus mindestens einem äußeren Deckpolymer besteht, das die Verstärkung an der Außenseite der Aufnahme bedeckt.

6. Kappe (1; 101; 201; 301) nach Anspruch 5, wobei die Hülle (2; 102; 202; 302) eine weitere Schicht umfasst, die aus mindestens einem inneren Deckpolymer besteht, das die Verstärkung an der Innenseite der Aufnahme bedeckt.

7. Kappe (1; 101; 201; 301) nach einem der vorstehenden Ansprüche, wobei die Wand der Hülle (2; 102; 202) nur aus einem oder mehreren Polymeren gebildet ist.

8. Kappe (1; 101; 201; 301) nach einem der vorstehenden Ansprüche, wobei das Befestigungsmittel eine Haltemanschette umfasst, die ein erstes Ende aufweist, das dazu bestimmt ist, sich am distalen Ende des Endoskops zu befestigen, wobei die Haltemanschette ein zweites Ende aufweist, das so an der Hülle befestigt ist, dass die Manschette in die proximale Öffnung mündet.

9. Kappe (1; 101; 201; 301) nach Anspruch 8, wobei die Manschette (3; 103; 203; 303) und die Wand der Hülle aus einem Stück gefertigt sind.

10. Kappe (1; 101; 201; 301) nach Anspruch 8 oder 9, wobei die Manschette (3; 103; 203; 303) aus einem Material besteht, das aus einem oder mehreren, als Polymere der Manschette bezeichneten Polymeren besteht.

11. Kappe (1; 101; 201; 301) nach Anspruch 10, wobei das Material, aus dem die Manschette (3; 103; 203; 303) besteht, elastisch und/oder porös ist.

12. Kappe (1; 101; 201; 301) nach einem der Ansprüche 8 bis 11, wobei die Manschette (3; 103; 203; 303) eine Innenfläche aufweist, die mindestens eine Vertiefung und/oder mindestens eine Erhebung umfasst.

13. Kappe (1; 201; 301) nach einem der vorstehenden Ansprüche, wobei die Wand eine Nachgiebigkeit aufweist, die es der Hülle (2; 202; 302) ermöglicht, in eine umgebogene Konfiguration gebracht zu werden, in der die Hülle um das Befestigungsmittel herum um sich selbst umgedreht ist, und durch Abrollen aus dieser Konfiguration in eine aufgebogene Konfiguration zu gelangen, die der Grundkonfiguration entspricht und in der die Hülle in die andere Richtung umgedreht ist, sodass sie neben dem Befestigungsmittel (3; 203; 303) angeordnet ist.

14. Kappe (1; 201; 301) nach Anspruch 13, wobei die Verstärkung erst im Bereich der Spitze (6) der Hülle (2; 202; 302) beginnt und sich ausschließlich in der Hülle befindet, wobei das Befestigungsmittel (3; 203; 303) frei von ihr ist, wobei die an die Verstärkung angrenzende Zone (303d) somit nachgiebiger ist als die Wand der Hülle und aufgrund dieser Tatsache ein Gelenk bildet, um das herum die Hülle um sich selbst in ihrer umgebogenen Konfiguration umgedreht werden kann.

15. Kappe (101) nach einem der Ansprüche 1 bis 12, wobei die Hülle (102) so angeordnet ist, dass sie durch Verformung aus einer kompaktierten Konfiguration in eine aufgebogene Konfiguration gelangen kann, die der Grundkonfiguration entspricht, wobei die Hülle in ihrer kompaktierten Konfiguration eine verringerte Aufweitung der Hülle in Bezug auf die Grundkonfiguration aufweist, oder keine Aufweitung aufweist, während gleichzeitig die der proximalen Öffnung (105) gegenüberliegende distale Öffnung (104) beibehalten wird.

16. Kappe (301) nach einem der vorstehenden Ansprüche, wobei die Wand (302'), die die Hülle (302) bildet, nicht durchgehend und auf ihrer gesamten Länge von einem proximalen Ende (303a) zu einem distalen Ende (303b) geschlitzt ist, sodass die Hülle (302) durch Einrollen der Wand (302') in der Längsrichtung um sich selbst erhalten werden kann, um die Hülle zwischen dem proximalen Ende (303a), das dazu bestimmt ist, sich um das Endoskop herum zu befinden, und dem distalen Ende (303b) zu schließen, das somit die Ränder der distalen Öffnung (304) bildet.

17. Verfahren zur Herstellung einer Kappe (1; 101; 201) für ein Endoskop (20, 120), wobei die Kappe einem der Ansprüche 1 bis 16 entspricht, wobei die Kappe eine Hülle (2; 102; 202) umfasst, die eine proximale Öffnung (5; 105) und eine der proximalen Öffnung gegenüberliegende distale Öffnung (4; 104) umfasst und eine Form einer Grundkonfiguration aufweist, wobei sich diese Form von ihrer proximalen Öffnung zu ihrer distalen Öffnung hin aufweitet, wobei das Herstellungsverfahren umfasst:
- einen Schritt des Ausführens und/oder Ausgestaltens einer Verstärkung (8) entweder in Form einer Manschette, deren Form mindestens einem Teil der Form der Grundkonfiguration entspricht, oder in Form eines Umhangs,
- einen Schritt des Bedeckens der Verstärkung mit einer Schicht, die aus mindestens einem Deckpolymer auf mindestens einer der Seiten der Verstärkung besteht,
wobei das Material der Verstärkung und/oder das oder die Deckpolymere einer Perforation und/oder einem Einschritt durch ein spitzes und/oder scharfkantiges Objekt standhalten, das Herstellungsverfahren.

## Claims

1. Endoscopic cap (1; 101; 201; 301) comprising:
- a protection envelope (2; 102; 202; 302) formed by a wall folded on itself such that said envelope comprises a proximal opening (5; 105; 305) and a distal opening (4; 104; 304) facing the proximal opening, and the wall having a shape tapered outwards from its proximal opening to its distal opening, in a basic configuration, wherein said envelope (2; 102; 202) defines a housing,
- attachment means (3; 103; 203; 303) arranged to enable attachment of the cap onto the distal end of an endoscope (20, 120) with said proximal opening (5; 105; 305) facing or around the distal end (21, 121) of the endoscope,
**characterised in that** the wall of said envelope is made of a structure with sufficient strength to resist perforation and/or incision by a pointed and/or cutting object, the wall of said envelope including a stiffener (8) arranged to surround this housing, said stiffener (8) being composed of one or several wires.

2. Cap (1; 101; 201; 301) according to claim 1, wherein said envelope (2; 102; 202; 302) forms a bell, the proximal opening (5; 105; 305) being arranged at the apex (6; 106) of this bell, and the distal opening (4; 104; 304) being arranged at the base (7; 107) of this bell.

3. Cap (1; 101; 201; 301) according to claim 1 or 2, wherein the stiffener can be composed of one or more wires made from a shape memory material.

4. Cap (1; 101; 201; 301) according to any one of the preceding claims, wherein the wire(s) form(s) a mesh.

5. Cap (1; 101; 201; 301) according to any one of the preceding claims, wherein said envelope (2; 102; 202; 302) includes at least one layer composed of at least one external cover polymer covering the stiffener at the exterior of said housing.

6. Cap (1; 101; 201; 301) according to claim 5, wherein said envelope (2; 102; 202; 302) includes at least one other layer composed of at least one internal cover polymer covering the stiffener at the interior of said housing.

7. Cap (1; 101; 201; 301) according to any one of the preceding claims, wherein the wall of said envelope (2; 102; 202) is formed from one polymer only or from several polymers.

8. Cap (1; 101; 201; 301) according to any one of the preceding claims, wherein the attachment means comprise a retention sleeve intended to be fixed on the distal end of the endoscope, the retention sleeve having a second end fixed on said envelope such that the sleeve opens up in said proximal opening.

9. Cap (1; 101; 201; 301) according to claim 8, wherein the sleeve (3; 103; 203; 303) and the wall of said envelope are a single piece made of the same material.

10. Cap (1; 101; 201; 301) according to claim 8 or 9, wherein the sleeve (3; 103; 203; 303) is composed of a material made of one or more polymers, called sleeve polymers.

11. Cap (1; 101; 201; 301) according to claim 10, wherein the material forming the sleeve (3; 103; 203; 303) is elastic and/or porous.

12. Cap (1; 101; 201; 301) according to any one of claims 8 to 11, wherein the sleeve (3; 103; 203; 303) has an internal surface comprising at least one recess and/or at least one relief.

13. Cap (1; 201; 301) according to any one of the preceding claims, wherein the wall is flexible so that the envelope (2; 202; 302) can be put into a folded configuration, in which the envelope is folded back on itself around the attachment means, and to change from this configuration to an unfolded configuration, corresponding to the basic configuration and in which said envelope is turned back in the other direction such that it is positioned adjacent to the attachment means (3; 203; 303).

14. Cap (1; 201; 301) according to claim 13, wherein the stiffener only begins at the apex (6) of the envelope (2; 202; 302) and remains exclusively in the envelope, the attachment means (3; 203; 303) not having a stiffener, the zone (303d) adjacent to the stiffener thus being more flexible than the wall of the envelope and thus forming a hinge around which the envelope can be turned back on itself in its folded configuration.

15. Cap (101) according to any one of claims 1 to 12, wherein the envelope (102) is arranged so as to pass, by deformation, from a compacted configuration to an expanded configuration corresponding to the basic configuration, in its compacted configuration the envelope having a smaller taper than in the basic configuration or not having a taper at all, while keeping the distal opening (104) facing the proximal opening (105).

16. Cap (301) according to any one of the preceding claims, wherein the wall (302') forming the envelope (302) is discontinuous and split over its entire length, from a proximal end (303a) to a distal end (303b), such that the envelope (302) can be obtained by rolling the wall (302') on itself along the direction of its length, so as to close the envelope between the proximal end (303a) intended to be around the endoscope, and the distal end (303b) that thus forms the edges of the distal opening (304).

17. Method of fabrication of a cap (1; 101; 201) for an endoscope (20, 120), said cap complying with any one of claims 1 to 16, said cap comprising an envelope (2; 102; 202) comprising a proximal opening (5; 105) and a distal opening (4 ; 104) facing the proximal opening and having a shape of a basic configuration, this shape tapering outwards from its proximal opening towards its distal opening, said fabrication method comprising:
- a step for fabrication and/or conformation of a stiffener (8) in the form of a handle for which the shape corresponds at least partly to the shape of the basic configuration, namely in the form of a cape,
- a step to cover the stiffener by a layer composed of at least one covering polymer on at least one of the stiffener faces,
the stiffener material and/or the covering polymer(s) being resistant to perforation and/or incision by a pointed and/or cutting object, said manufacturing method.
